# EUROPEAN PATENT SPECIFICATION

(11) **EP 1 727 524 B1**
(45) Date of publication and mention of the grant of the patent: **21.10.2009**
(21) Application number: 04721911.8
(22) Date of filing: 19.03.2004
(51) Int. Cl.: A61K 9/50, A61K 31/545

(54) **PREPARATION OF LIPID COATED CEFUROXIME AXETIL**
HERSTELLUNG VON LIPID-BESCHICHTETEM CEFUROXIME AXETIL
PREPARATION DE CEFUROXIME AXETIL A ENROBAGE LIPIDIQUE

(43) Date of publication of application: 06.12.2006
(73) Proprietor: Zentiva Kimyasal Ürünler Sanayi ve Ticaret A.S., Levent Istanbul 34394 (TR)
(72) Inventor: DABAK, Kadir, Levent, 34394 Istanbul (TR); GÖREN, Adil, Levent, 34394 Istanbul (TR); ULUSOY, Cumhur, Levent, 34394 Istanbul (TR); ÖNER, Timucin, Levent, 34394 Istanbul (TR); SÖNMEZ, Seyfettin, Levent, 34394 Istanbul (TR); PEMBECIOGLU, Gürkan, Çerkezköy 59500 Tekirdag (TR); TANRIKULU, Armagan, Çerkezköy 59500 Terkirdag (TR); URAS, Ibrahim Alper, Levent, 34394 Istanbul (TR); TAHIR, Refiye, Levent, 34394 Istanbul (TR); ILERI, Vahdet, Levent, 34394 Istanbul (TR); BARIN, Ibrahim, Levent, 34394 Istanbul (TR); DEMIREL, Mete, Levent, Istanbul, 34394 (RE); KANBUR, Selim, Levent, 34394 Istanbul (TR); SUCUOGLU, Semih, Levent, 34394 Istanbul (TR); AKYÜZ, Yunus, Çerkezköy 59500 Terkirdag (TR); PEMBECIOGLU, Ece, Çerkezköy 59500 Terkirdag (TR)
(74) Representative: Sevinç, Erkan
(86) International application number: PCT/EP2004/050336
(87) International publication number: WO 2005/094791

(56) References cited:
- WO-A-01/37808
- WO-A-02/36126
- US-A- 4 865 851
- US-A1- 2003 170 310

## Description

### Summary of the Invention

The present invention relates a novel process for coating of cefuroxime axetil with lipid or a mixture of lipids to mask the bitter taste of cefuroxime axetil. The novel process comprises the steps of atomizing the molten lipid or a mixture of lipids on a gas (preferably air) mixing particulate cefuroxime axetil, solidification of the molten lipid or mixture of lipids on the particulate cefuroxime axetil and recovering said lipid or mixture of lipids coated cefuroxime axetil particles. The molten lipid solidifies upon cooling and coats the particles which are maintained in suspension in a fluid bed.

### Background of the Invention

Cefuroxime axetil is a semisynthetic, broad-spectrum cephalosporin antibiotic for oral administration. Chemically, cefuroxime axetil, the 1-(acetyloxy) ethyl ester of cefuroxime, is (*RS*)-1-hydroxyethyl (6*R*,7*R*)-7-[2-(2-furyl)glyoxylamido]-3-(hydroxymethyl)-8-oxo-5-thia-1-azabicydo[4.2.0]oct-2-ene-2-carboxylate, 7²-(Z)-(O-methyl-oxime),1 -acetate 3-carbamate. Its molecular formula is C₂₀H₂₂N₄O₁₀S, having a molecular weight of 510.48.

Cefuroxime, as disclosed in GB 1453049, is a valuable broad spectrum antibiotic. Preparation of cefuroxime is described in US 3974153. 1-acetoxyethyl ester of cefuroxime which is known as cefuroxime axetil improves the effectiveness on oral administration as disclosed in GB 1571683. Preparation of cefuroxime axetil is described in DE 2706413.

For ease and safety of administration, most of the drugs are formulated as tablets or capsules for oral administration. However, some patients such as children and the elderly, often experience difficulty in swallowing solid oral dosage forms. For these patients, drugs are commonly provided in liquid dosage forms such as solutions, emulsions and suspensions. Solutions or suspensions of granules as, for example, a syrup are particularly convenient for oral administration of antibiotics to children. However, cefuroxime axetil has an extremely bitter taste which is long lasting and which cannot be adequately masked by the addition of sweeteners or flavors to conventional granule presentation. Another problem arises from the tendency of cefuroxime axetil, both in crystalline form and the amorphous form to form a gelatinous mass when contacted with aqueous media. Such gel formation would lead to the poor dissolution of the cefuroxime axetil and hence poor absorption from the gastrointestinal tract.

In the formulation of cefuroxime axetil into granules it is important to avoid the release of the drug into any liquid medium in which it is suspended or indeed into the mouth when administering. Such problems may be minimized by formulating the cefuroxime axetil as lipid coated particles. In order to improve the properties of the suspension, as described in GB 2204792, cefuroxime axetil is coated with stearic acid. This wax coated product is known as stearic acid-coated cefuroxime axetil or shortly, SACA. GB 2204792 discloses a particulate formulation in which above referenced problems are addressed. This patent discloses a composition comprising cefuroxime axetil in particulate form, the particles being provided with integral coatings of a lipid or a mixture of lipids which are insoluble in water and which serve to mask the bitter taste of cefuroxime axetil upon oral administration but which disperse or dissolve on contact with gastrointestinal fluid. The formulated coated particles break down upon contact with gastrointestinal fluid, thus allowing rapid dispersion and dissolution in the gastrointestinal tract.

In GB 2204792, it is described that, the coated particles of the invention may conveniently be prepared by atomizing a dispersion of particulate cefuroxime axetil in a molten lipid and cooling the coated particles. The dispersion may be prepared by adding particulate cefuroxime axetil to the molten lipid or mixture of lipids or alternatively mixing the ingredients of the dispersion together in the solid state and then melting the lipid or mixture of lipids. The particulate cefuroxime axetil can be dispersed in the molten lipid using conventional techniques, for example, using a high shear mixer. Generally, the temperature of the molten lipid will be 10 °C to 20 °C above its melting point. The molten dispersion is atomized to give particles of lipid-coated cefuroxime axetil on cooling. The coated particles may be solidified and collected by conventional techniques. The coated particles may conveniently be solidified by applying a stream of cool air or preferably dry nitrogen to the spray chamber at a temperature of for example 0 °C to 30 °C, preferably 5 °C -20 °C such that cooling and solidification of the particles is complete. This process can be defined as a spray coating system.

### Objects of the Invention

One of the objects of the present invention is to provide a novel process for coating of cefuroxime axetil with a lipid or a mixture of lipids to mask the bitter taste of cefuroxime axetil in oral administration.

Another object of the present invention is to provide a process in which cefuroxime axetil is exposed to less heat compared with conventional processes, thereby eliminating the risk of impurity formation.

Another object of the present invention is to provide a simple process in which the need for mixing a semi-solid dispersion is avoided to shorten the overall production time and to increase unit production per time.

Yet another object of the present invention is to provide a fast, reliable and cheap process for coating of cefuroxime axetil, which process is free of product decomposition risk.

### Detailed Description of the Invention

The present invention proposes a process for coating of cefuroxime axetil with a lipid or a mixture of lipids to mask the bitter taste of cefuroxime axetil, the process comprising the steps of atomizing the molten lipid or the mixture of lipids on preferably air-mixing particulate cefuroxime axetil, solidification of the molten lipid or mixture of lipids on the particulate cefuroxime axetil, and recovering said lipid or mixture of lipids coated cefuroxime axetil particles. The molten lipid solidifies upon cooling and coats the particles which are maintained in suspension in a fluid bed. Advantageously, it has been found that cefuroxime axetil is exposed to less heat compared with the process described in GB 2204792, which proposes a process in which cefuroxime axetil is added to molten lipid, mixed together and atomized to a chamber. During this process, cefuroxime axetil is exposed to heat of the molten lipid. Apparently, this process needs additional care to avoid impurity formation. Additionally, a very careful mixing and thorough stirring as well as homogenous atomization are needed to have homogenous molten lipid-cefuroxime axetil dispersion.

The process of the present invention requires that the molten lipid or the mixture of lipids is atomized on a gas (preferably air) mixing particulate cefuroxime axetil which has a temperature of between 0 °C - 40 °C, preferably at 20 °C - 25 °C. A molten lipid or a mixture of lipids is solidified on the particulate cefuroxime axetil and cefuroxime axetil particles which are coated with the lipid or mixture of lipids are recovered. This process can be defined as a simple fluidized bed coating system. The present invention provides a fast, reliable, cheap and a low product decomposition risky process.

The composition of the invention may contain cefuroxime axetil in crystalline form or more preferably in amorphous form, with a mean particle diameter by volume in the range 5-50 microns.

In order to provide taste-masked particles of cefuroxime axetil suitable for oral administration, the melting point of the lipid used should be sufficiently high to prevent melting of the coated particles in the mouth, thereby leading to release of the bitter tasting active ingredient, but not so high that the cefuroxime axetil active ingredient itself melts and/or becomes chemically degraded during the coating process. Thus, the lipid or mixture of lipids for use in the present invention has a melting point of from 30 °C to 80 °C and preferably from 40 °C to 70 °C. Where the composition according to the invention contains amorphous cefuroxime axetil, the melting point of the lipid or mixture of lipids is still more preferably in the range of 45 °C to 60 °C.

Suitable lipids which can be used in our process include fatty acids or monohydric alcohols thereof, fixed oils, fats, waxes, sterols, phospholipids and glycolipids. The lipid may, for example, be of a high molecular weight (C10-30) straight chain saturated or unsaturated aliphatic acid, such as stearic acid or palmitic acid; a triglyceride for example a glyceryl ester of a high molecular weight (C10-30) aliphatic acid, such as glyceryl trilaurate or glyceryl trimyristate; a partially hydrogenated vegetable oil such as cottonseed oil or soyabean oil; a wax for example beeswax or carnauba wax; a high molecular weight (C10-30) straight chain aliphatic alcohol such as stearyl alcohol or cetyl alcohol; or a mixture thereof. Mixtures of high molecular weight fatty acids such as mixtures of stearic and palmitic acids, mixtures of high molecular weight straight chain aliphatic alcohols, such as cetostearyl alcohol, mixtures of partially hydrogenated cottonseed and soyabean oils and mixtures of high molecular weight aliphatic acids and glyceryl esters such as a mixture of stearic acid and glyceryl trilaurate may, for example, can be used.

The lipid coated particles according to the invention will preferably contain from 5 to 90%, more preferably from 5 to 50% and still more preferably from 5 or 10 to 30% by weight of cefuroxime axetil.

It is known that, particles provided with an integral lipid coating to mask the bitter taste of the cefuroxime axetil can have a diameter of less than 250 microns. Coated particles with diameters in the range of from 1 to 250 microns are thus preferred. The size of the coated particles is an important factor with respect to the bioavailability of the cefuroxime axetil and the acceptability of such products for oral administration, average particle sizes in excess of about 250 microns mean diameter by volume giving an undesirable gritty taste.

A particularly preferred dispersion for preparing lipid coated particles of cefuroxime axetil is a dispersion of cefuroxime axetil in a mixture of stearic acid and palmitic acid in a ratio in the range 3:7 to 7:3 by weight, preferably in the ratio of about 1:1 by weight. The amount of cefuroxime axetil in the dispersions for preparing lipid-coated particles according to the present invention is calculated to provide the desired amount of cefuroxime axetil in the coated particle as discussed above.

Stearic acid is specified as a mixture of fatty acids, chiefly stearic and palmitic acids in the British Pharmaceutical Codex (1973). Particle size measurements for Examples 1 to 4 were made by Malvern Mastersizer-2000 Laser Diffraction Particle Size Analyzer. The following Examples illustrate the invention.

### Example 1

Stearic acid (450 g) was melted by raising the temperature of the lipid to a temperature of about 15 °C above its melting point and fed into the fluidized bed coating apparatus using a peristaltic pump and two nozzles while amorphous cefuroxime axetil is air mixed. The product was chilled using a stream of air fed into the fluid bed chamber at the temperature indicated in Table 1 below and the solidified product was collected at the end of the process.

| **Table -1 : Process Parameters** | | |
|---|---|---|
| Air Volume (m³/h) | | 190 |
| Air Temperature (°C) | | 25 |
| Product Temperature (°C) | | 22 |
| Nozzles Diameter (mm) | | 0.8, 0.8 |
| Spray Pressure (bar) | | 1 |
| Spray Temperature (°C) | | 150 |
| Microclimate Pressure (bar) | | 0.5 |
| Microclimate Temperature (°C) | | 85 |
| Feed Rate (g/min) | | 37 |
| Obtained particle size (µm) | | |
| | d(0.1) | 14 |
| | d(0.5) | 103 |
| | d(0.9) | 222 |

### Example 2

The procedure of Example 1 was used while utilizing process parameters of Table 2 below:

| **Table -2 : Process Parameters** | | |
|---|---|---|
| Air Volume (m³/h) | | 190 |
| Air Temperature (°C) | | 25 |
| Product Temperature (°C) | | 22 |
| Nozzle Diameter (mm) | | 0.8, 0.8 |
| Spray Pressure (bar) | | 1 |
| Spray Temperature (°C) | | 150 |
| Microclimate Pressure (bar) | | 0.5 |
| Microclimate Temperature (°C) | | 85 |
| Feed Rate (g/min) | | 24 |
| Obtained particle size (µm) | | |
| | d(0.1) | 23 |
| | d(0.5) | 73 |
| | d(0.9) | 171 |

### Example 3

The procedure of Example 1 was used while utilizing following process parameters of Table 3 below:

| **Table -3 : Process Parameters** | | |
|---|---|---|
| Air Volume (m³/h) | | 190 |
| Air Temperature (°C) | | 25 |
| Product Temperature (°C) | | 22 |
| Nozzle Diameter (mm) | | 0.8, 0.8 |
| Spray Pressure (bar) | | 1 |
| Spray Temperature (°C) | | 150 |
| Microclimate Pressure (bar) | | 0.5 |
| Microclimate Temperature (°C) | | 85 |
| Feed Rate (g/min) | | 20 |
| Obtained particle size (µm) | | |
| | d(0.1) | 19 |
| | d(0.5) | 60 |
| | d(0.9) | 150 |

### Example 4

The procedure of Example 1 was used while utilizing following process parameters of Table 4 below:

| **Table -4 : Process Parameters** | | |
|---|---|---|
| Air Volume (m³/h) | | 190 |
| Air Temperature (°C) | | 25 |
| Product Temperature (°C) | | 22 |
| Nozzle Diameter (mm) | | 0.8, 0.8 |
| Spray Pressure (bar) | | 1 |
| Spray Temperature (°C) | | 150 |
| Microclimate Pressure (bar) | | 0.5 |
| Microclimate Temperature (°C) | | 85 |
| Feed Rate (g/min) | | 16 |
| Obtained particle size (µm) | | |
| | d(0.1) | 13 |
| | d(0.5) | 43 |
| | d(0.9) | 108 |

## Claims

1. A process for coating a lipid or a mixture of lipids onto cefuroxime axetil, comprising the steps of:
a) charging cefuroxime axetil in particulate form into a fluid bed dryer,
b) atomizing a molten lipid or a mixture of lipids on cefuroxime axetil in particulate form,
c) solidifying said molten lipid or the mixture of lipids on the particulate cefuroxime axetil,
d) recovering said lipid or mixture of lipids coated cefuroxime axetil particles.

2. A process as set forth in claim 1 wherein the lipid or mixture of lipids comprises one or more straight chain aliphatic carboxylic acids having from 10 to 30 carbon atoms.

3. A process as set forth in claim 1 wherein the lipid or mixture of lipids has a melting point in the range of 30 °C to 80 °C.

4. A process as set forth in claim 1 wherein the mixture of lipids comprises a mixture of stearic and palmitic acids in a ratio of from 3:7 to 7:3 by weight.

5. A process as set forth in claim 4 wherein the mixture of stearic and palmitic acids is in a ratio of about 1:1 by weight.

6. A process as set forth in any of the preceding claims wherein the lipid or mixture of lipids coated cefuroxime axetil particles contain from 5 to 90% by weight of cefuroxime axetil.

7. A process as set forth in claim 1 wherein the particulate cefuroxime axetil is amorphous cefuroxime axetil.

8. A process as set forth in any of the preceding claims wherein the coated particles of cefuroxime axetil have diameters in the range of 1 to 250 microns.

9. A process as claimed in claim 1 wherein the molten lipid or the mixture of lipids is atomized on cefuroxime axetil by a nozze atomizer using peristaltic pump.

10. A process as claimed in claim 1 wherein the molten lipid is atomized at a temperature which is in the range of 10°C to 20°C above the melting point of the lipid or mixture of lipids used.

11. A process as claimed in any of the preceding claims wherein the gas medium into which cefuroxime axetil is charged in particulate form is air.

## Patentansprüche

1. Verfahren zur Beschichtung eines Lipids oder einer Lipidmischung auf Cefuroximaxetil, umfassend die Schritte:
a) Füllen des Cefuroximaxetil in Teilchenform in einen Wirbelschichttrockner,
b) Zerstäuben eines geschmolzenen Lipids oder einer Lipidmischung auf Cefuroximaxetil in Teilchenform,
c) Verfestigen des genannten geschmolzenen Lipids oder der Lipidmischung auf dem teilchenförmigen Cefuroximaxetil,
d) Gewinnen der genannten, mit dem Lipid oder der Lipidmischung beschichteten, Cefuroximaxetilteilchen.

2. Verfahren nach Anspruch 1, wobei das Lipid oder die Lipidmischung eine oder mehrere geradkettige aliphatische Carbonsäuren, die von 10 bis 30 Kohlenstoffatome haben, umfasst.

3. Verfahren nach Anspruch 1, wobei das Lipid oder die Lipidmischung einen Schmelzpunkt in dem Bereich von 30°C bis 80°C hat.

4. Verfahren nach Anspruch 1, wobei die Lipidmischung eine Mischung aus Stearin- und Palmetinsäure in einem Gewichtsverhältnis von 3:7 zu 7:3 umfasst.

5. Verfahren nach Anspruch 4, wobei die Mischung aus Stearin- und Palmetinsäure in einem Gewichtsverhältnis von etwa 1:1 liegt.

6. Verfahren nach irgendeinem der vorangegangenen Ansprüche, wobei die mit dem Lipid oder mit der Lipidmischung beschichteten Cefuroximaxetilteilchen von 5 bis 90 Gew.-% Cefuroximaxetil enthalten.

7. Verfahren nach Anspruch 1, wobei das teilchenförmige Cefuroximaxetil amorphes Cefuroximaxetil ist.

8. Verfahren nach irgendeinem der vorangegangenen Ansprüche, wobei die beschichteten Cefuroximaxetilteilchen einen Durchmesser in dem Bereich von 1 bis 250 Mm haben.

9. Verfahren beansprucht wie in Anspruch 1, wobei das geschmolzene Lipid oder die Lipidmischung auf Cefuroximaxetil drauf mittels einer Zerstäuberdüse, die eine peristaltische Pumpe verwendet, zerstäubt wird.

10. Verfahren beansprucht wie in Anspruch 1, wobei das geschmolzene Lipid bei einer Temperatur, die in dem Bereich von 10°C bis 20°C über dem Schmelzpunkt des verwendeten Lipids oder der Lipidmischung liegt, zerstäubt wird.

11. Verfahren beansprucht wie in irgendeinem der vorangehenden Ansprüche, wobei das Gasmedium, in dem das Cefuroximaxetil in Teilchenform eingefüllt wird, Luft ist.

## Revendications

1. Un procédé d'enrobage d'un lipide ou d'un mélange de lipides sur le céfuroxime axétil comprenant les étapes suivantes:
- Chargement du céfuroxime axétil sous forme particulaire dans un sécheur à lit fluidisé ;
- l'atomisation d'un lipide en fusion ou d'un mélange de lipides sur le céfuroxime axétil sous forme particulaire ;
- la solidification dudit lipide en fusion ou du mélange de lipides sur le céfuroxime axétil particulaire ;
- la récupération dudit lipide ou dudit mélange de lipides enrobant les particules de céfuroxime axétil.

2. Un procédé selon la revendication 1, dans lequel le lipide ou le mélange de lipides comporte une ou plusieurs chaînes droites d'acides carboxyliques aliphatiques ayant de 10 à 30 atomes de carbone.

3. Un procédé selon la revendication 1, dans lequel le lipide ou le mélange de lipides a un point de fusion de l'ordre de 30C à 80C.

4. Un procédé selon la revendication 1, dans lequel le mélange de lipides comporte un mélange d'acides stéariques et palmitiques dans une proportion de 3:7 à 7:3 en poids.

5. Un procédé selon la revendication 4, dans lequel le mélange d'acides stéariques et palmitiques est dans une proportion d'environ 1:1 en poids.

6. Un procédé tel qu'exposé dans l'une des revendications précédentes, dans lequel le lipide ou le mélange de lipides enrobant les particules de céfuroxime axétil contient de 5 à 90 % en poids de céfuroxime axétil.

7. Un procédé selon la revendication 1, dans lequel le céfuroxime axétil particulaire est un céfuroxime axétil amorphe.

8. Un procédé tel qu'exposé dans l'une des revendications précédentes, dans lequel les particules de céfuroxime axétil enrobées ont un diamètre de l'ordre de 1 à 250 microns.

9. Un procédé selon la revendication 1, dans lequel le lipide en fusion ou le mélange de lipides est atomisé sur le céfuroxime axétil par un buse atomiseur utilisant une pompe péristaltique.

10. Un procédé selon la revendication 1, dans lequel le lipide en fusion est atomisé à une température qui est de l'ordre de 10 C à 20 C au-dessus du point de fusion du lipide ou du mélange de lipides utilisé.

11. Un procédé tel que revendiqué dans l'une des revendications précédentes, dans lequel le milieu gazeux dans lequel le céfuroxime axétil est chargé sous forme particulaire est de l'air.
